# EUROPÄISCHE PATENTSCHRIFT

(11) **EP 0 649 654 B1**
(45) Veröffentlichungstag und Bekanntmachung des Hinweises auf die Patenterteilung: **10.02.1999**
(21) Anmeldenummer: 94117179.5
(22) Anmeldetag: 21.11.1991
(51) Int. Cl.: A61K 31/40, A61K 31/275, A61K 38/55

(54) **Verwendung einer Kombination aus Angiotensin-Converting-Enzyme-Hemmer und Calciumantagonist zur Behandlung einer Proteinurie**
Use of a combination of an ACE-inhibitor with a calcium antagonist in the treatment of proteinuria
Utilisation d'une association d'un inhibiteur ace avec un antagoniste du calcium pour traiter la protéinurie

(30) Priorität: 27.11.1990 DE 4037691
(43) Veröffentlichungstag der Anmeldung: 26.04.1995
(62) Teilanmeldung aus: 91119892.7
(73) Patentinhaber: HOECHST AKTIENGESELLSCHAFT, 65929 Frankfurt am Main (DE)
(72) Erfinder: Becker, Reinhard, Dr., D-65189 Wiesbaden (DE); Henning, Rainer, Dr., D-65795 Hattersheim (DE); Teetz, Volker, Dr., D-65719 Hofheim (DE); Urbach, Hansjörg, Dr., D-61476 Kronberg (DE)

(56) Entgegenhaltungen:
- EP-A- 0 265 685
- EP-A- 0 381 075
- EP-A- 0 508 511
- ARCHIVES OF PHARMACOLOGY, vol.349, no. SUPP, 1994, page A248; K. JOCHIMS ET AL.: 'NEPHROPROTECTIVE EFFECT OF THE COMBINATION VERAPAMIL/TRANDOLAPRIL IN HYPERTENSIVE RATS'

## Beschreibung

Die vorliegende Erfindung betrifft die Prävention und Therapie einer Proteinurie durch kombinierte Gabe eines Angiotensin-Converting-Hemmers (ACE-Hemmer) und eines Calciumantagonisten.

ACE-Hemmer sind Verbindungen, welche die Umwandlung von Angiotensin I in das pressorisch wirksame Angiotensin II verhindern. Solche Verbindungen sind beispielsweise in den folgenden Patentanmeldungen beschrieben: US-PS 4 350 633, US-PS 4 344 949, US-PS 4 294 832, US-PS 4 350 704, EP-A 31 741, EP-A 51020, EP-A 49658, EP-A 29488, EP-A 46953, EP-A 52870, US-P 4 129 571, US-PS 4 154 960, US-PS 4 374 829, EP-A 79522, EP-A 79022, EP-A 51 301, US-PS 4 454 292, US-PS 4 374 847, EP-A 72352, EP-A 84164, US-PS 4 470 972, EP-A 65301 und EP-A 52991. Ihr blutdrucksenkende Wirkung ist gut dokumentiert.

Calciumantagonisten sind solche Verbindungen, die den Einstrom von Calcium-Ionen in Zellen, insbesondere glatte Muskelzellen beeinflussen. Solche Verbindungen sowie ihre blutdrucksenkende Wirkung sind in einer Vielzahl von Publikationen und Patentanmeldungen niedergelegt.

Kombinationen von ACE-Hemmern und Calciumantagonisten und deren Verwendung bei der Behandlung des Bluthochdrucks sind beispielsweise bekannt aus EP-A 180 785 und EP-A 265 685.

Die blutdrucksenkende Wirkung nach kombinierter Gabe von ACE-Hemmern und Calciumantagonisten wird auch von D.Klaus [Z.Kardiol, Band 77 (Suppl. 3), 1988, S. 73-88] und J. B. Kostis [Am. Heart J. (1988) Band 116, Nr. 6/1, 1988, S. 1591-1605] beschrieben.

Die kombinierte Gabe von ACE-Hemmern und Calciumantagonisten bei der Behandlung einer Arzneimittelabhängigkeit ist aus EP-A 381 075 bekannt.

Eine Proteinurie tritt auf insbesondere bei Erkrankungen des Nierengewebes (Nephritis, Nephrose, Schrumpfniere) und bei Stauungsniere durch Herzinsuffizienz; außer den Albuminen gelangen auch die Globuline und andere Buteiweißkörper in den Harn. Die höchsten Grade der Proteinurie finden sich bei Nephrose und Amyloidose. Eine konstante Proeinurie bei Diabetes mellitus weist auf die Entwicklung einer Glomerulosklerose (Kimmelstiel-Wilson' Syndrom) hin.

Eine Proteinurie-reduzierende Wirkung einiger ACE-Hemmer ist beschrieben [B. Kostis, Am. Heart J. (1988) Band 116, Nr. 6/1 S. 1591-1605; Z. Allgemeinmed. (1990) Band 66 (32), 924].

Studien an diabetischen Ratten zeigten, daß hingegen Calciumantagonisten eine Proteinurie nicht verbessern bzw. zu einer deutlichen Verschlechterung der Proteinurie führen. [B. Jackson et al., (1987), J. Cardiovasc. Pharmacology, 10 (Suppl 10), S167-S169].

Es wurde nun überraschenderweise gefunden, daß sich eine Kombination aus ACE-Hemmer und Calciumantagonist zur Präventation und Therapie einer Proteinurie eignet, wie diese typischerweise auftreten kann bei Diabetes mellitus und Nierenmassenverlust (Spenderniere), aber auch als Folgeerscheinung einer Glomerulosklerose infolge von Hyperperfusion der Glomeruli.

Von ganz besonderen Interesse ist die Kombination Trandolapril + Verapamil oder die physiologisch verträglichen Salze der genannten Einzelkomponenten, soweit diese Salze bilden.

Neben der Anwendung der genannten Kombination betrifft die Erfindung auch die gleichzeitige, getrennte oder zeitlich abgestufte Anwendung von ACE-Hemmern und Calciumantagonisten bei der Behandlung der Proteinurie.

Die pharmazeutischen Zubereitungen können beispielsweise hergestellt werden, indem man entweder die Einzelkomponenten als Pulver intensiv mischt, oder indem man die Einzelkomponenten in einem geeigneten Lösungsmittel wie beispielsweise einem niederen Alkohol auflöst und das Lösungsmittel anschließend entfernt.

Das Verhältnis der Wirkstoffe in den erfindungsgemäßen Kombinationen und Zubereitungen beträgt vorzugsweise 1-15 Gewichtsteile ACE-Hemmer zu 15-1 Gewichtsteilen Calciumantagonist. Die erfindungsgemäßen Kombinationen und Zubereitungen enthalten insgesamt vorzugsweise 0,5-99,5 Gew.-%, insbesondere 4-96 Gew.-% dieser Wirkstoffe.

Bei der erfindungsgemäßen Anwendung bei Säugern, vorzugsweise beim Menschen, bewegen sich beispielsweise die Dosen eines ACE-Hemmers der o .g. Formel im Bereich von 0,05 bis 100 mg/kg/Tag und die eines Calciumantagonisten im Bereich von 0,05 bis 200 mg/kg/Tag.

Die erfindungsgemäßen Zubereitungen bzw. Erzeugnisse können parenteral oder oral verabreicht werden. Bevorzugt ist die orale Applikationsform.

Die pharmakologisch verwendbaren Kombinationen der vorliegenden Erfindung und ihre Salze können zur Herstellung von pharmazeutischen Präparaten verwendet werden, welche eine wirksame Menge der Aktivsubstanzen zusammen mit Trägerstoffen enthalten und die sich zur enteralen und parenteralen Verabreichung eignen. Vorzugsweise verwendet werden Tabletten oder Gelatinekapseln, welche die Wirkstoffe zusammen mit Verdünnungsmitteln, z.B. Lactose, Dextrose, Rohrzucker, Mannitol, Sorbitol, Cellulose und/oder Glycerin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine, Traganth, Methylcellulose, Natriumcarboxymethylcellulose und/oder Polyvinylpyrrolidon, und, falls benötigt, Farbstoff, Geschmacksstoffe und Süßmittel.

Injizierbare Lösungen sind vorzugsweise isotonische wäßrige Lösungen oder Suspensionen, die sterilisiert sein können.

Als Salze der obengenannten Verbindungen kommen, je nach sauerer oder basischer Natur dieser Verbindungen, Alkali- oder Erdalkalisalze oder Salze mit physiologisch verträglichen Aminen oder Salze mit anorganischen oder organischen Säuren wie z.B. HCl, HBr, H₂SO₄, Maleinsäure, Fumarsäure, Weinsäure, Citronensäure in Frage.

### Referenzbeispiel:

### Präventation einer progressiven Proteinurie in Ratten mit Restniere

### 1. Methode

Männlichen ausgewachsenen Sprague-Dawley Ratten wurden zunächst 2/3 der rechten Niere durch Ligatur infarziert und im Abstand von einer Woche die kontralaterale Niere entfernt. Die Tiere erhielten unmittelbar danach über 8 Wochen die Medikation mit dem Futter. Aus dem Futterverbrauch ergab sich, daß Gruppe II (n:17) mit 58 mg/kg Felodipin (FE), Gruppe III (n:10) mit 1,6 mg/kg Ramipril (RA) und Gruppe IV (n:11) mit 41 mg/kg FE und 1,4 mg/kg RA behandelt wurden. Die Kontrollgruppe I erhielt Normalfutter. Der Urin wurde über jeweils 24 Stunden vor den Operationen und nach 1,3, 5 und 7 Wochen gesammelt. Blutproben wurden aus dem retroorbitalen Plexus unter leichter Pentobarbitalnarkose jeweils am Tag zuvor und am Ende der Studie nach der Blutdruckmessung in der A. Karotis in finaler Pentobarbitalnarkose gezogen.

### 2. Ergebnisse

Der mittlere arterielle Blutdruck betrug 160 ± 7 mm Hg in der Kontrollgruppe I, 122±3 mm Hg unter FE, 117±7 mm Hg unter RA und 108±4 mm Hg unter FE und RA. Die Proteinurie, die weniger als 20 mg/24 h vor der Operation betrug, vergrößerte sich in der Kontrollgruppe kontinuierlich bis auf 105±28 mg/24 h in der 7. Woche. Die Zunahme der Proteinurie verzögerte sich unter FE um 2 Wochen erreichte aber letzlich mit 114±28 mg/24 h ähnliche Werte. Mit 48±15 mg/24 h war die Proteinausscheidung unter RA deutlich geringer. Sie war nochmals auf nur 31±4 mg/24 h unter RA plus FE reduziert. Die Plasmakreatininspiegel verdoppelten sich nach der Operation in allen Gruppen von im Mittel 42 auf 73 mol/l, wurden aber danach durch die Medikation nicht weiter beeinflußt. Die Plasma-CE-Aktivität wurde unter RA mit und ohne FE um im Mittel 80% vermindert während FE allein keinen Einluß hatte.

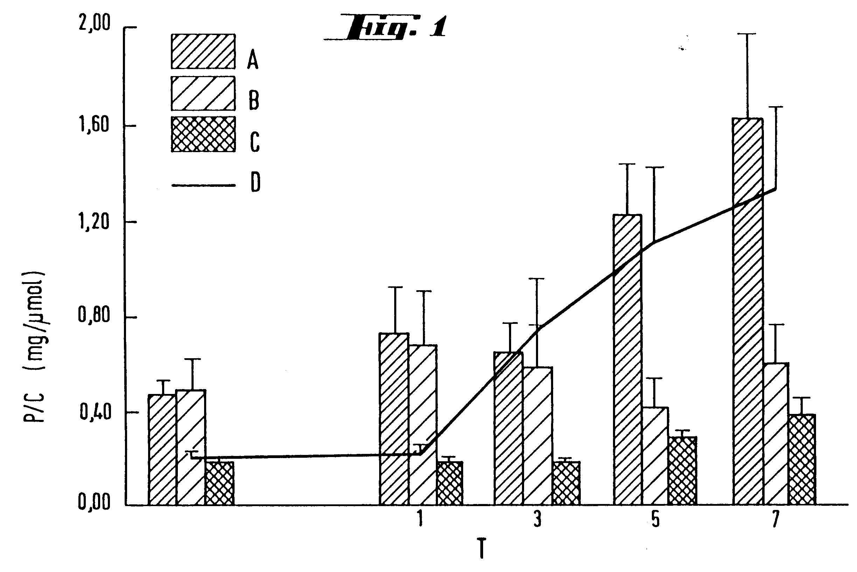

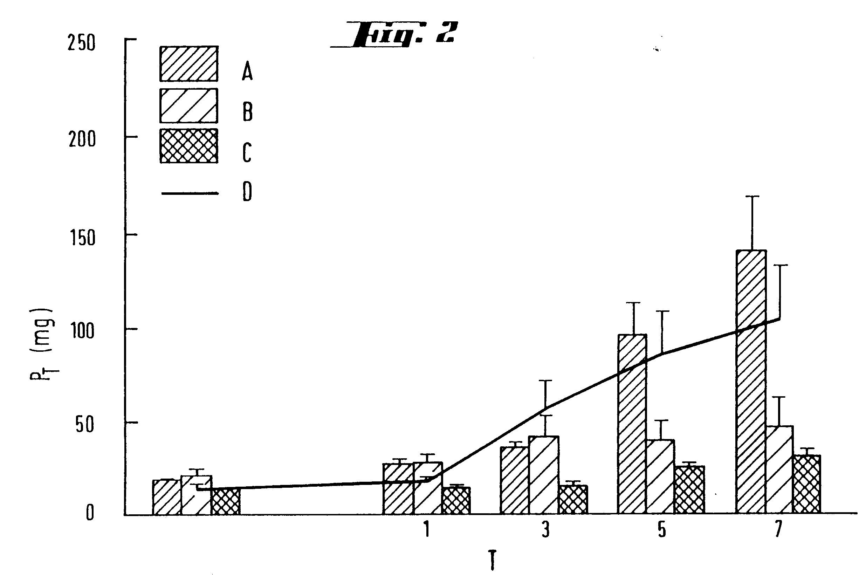

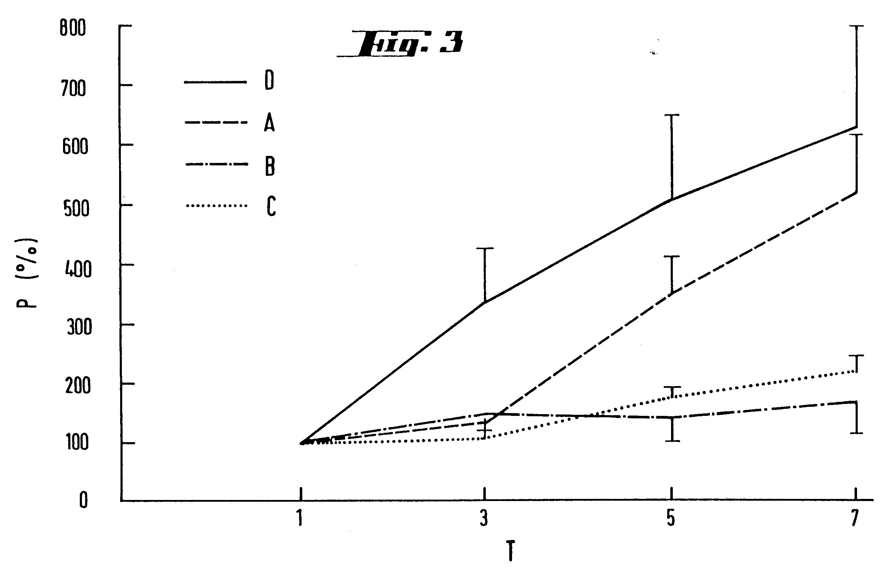

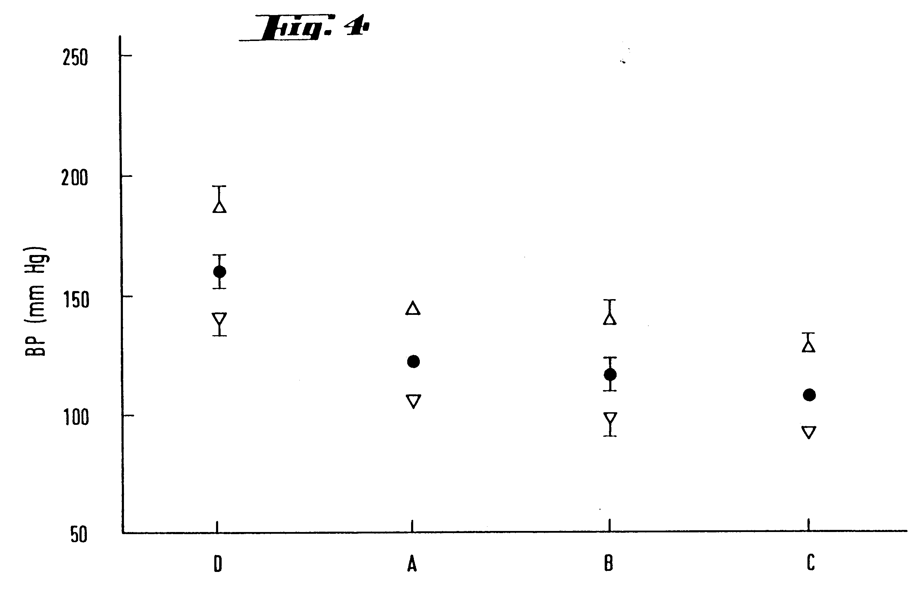

In Figur 1 und 2 ist T die Versuchsdauer in Wochen; die linken Werte ohne Zahl sind vor der Operation. Die in Figur 1-4 mit A bezeichneten Werte sind nach der Gabe von 58 mg/kg Felodipin, jene mit B nach Gabe von 1,6 mg/kg Ramipril, jene mit C nach Gabe einer Kombination von 1,4 mg Ramipril und 41 mg Felodipin gemessen. D stellt die Kontrolle dar.
Figur 1 zeigt das Verhältnis aus Protein-Ausscheidung und Kreatinin-Ausscheidung P/C in mg/µmol.
Figur 2 zeigt die Protein-Gesamtausscheidung P_{T} (mg) während 24 Stunden.
In Figur 3 ist die prozentuale Protein-Ausscheidung %P während 24 Stunden gegen die Versuchsdauer T aufgetragen.
Figur 4 zeigt den Blutdruck BP (mm Hg), wobei ● der mittlere Blutdruck, △ der systolische Blutdruck und ∇ der diastolische Blutdruck ist.

### 3. Diskussion

Ergebnisse von Versuchen mit Ratten belegen, daß Calciumantagonisten und ACE-Hemmer trotz gleicher Wirkung auf den systemischen Blutdruck in ihrer Wirkung auf die durch Verlust von Nephronen bedingte Verschlechterung der Nierenfunktion divergieren.

Die Untersuchung ergab weiter, daß eine Kontrolle des systemischen Blutdruckes mit einem Calciumantagonisten zwar den Beginn der Proteinurie verzögert, letztlich aber eher eine Beschleunigung des Nierenfunktionsverlustes bewirkt. Diese ungünstige Entwicklung konnte durch gleichzeitige Gabe eines ACE-Hemmers aufgehoben werden.

Die folgenden Beispiele dienen zur Erläuterung der vorliegenden Erfindung, ohne daß diese darauf beschränkt wäre:

### Beispiel 1:

Herstellung eines oralen Kombinationspräparates aus Trandolapril und Verapamil 1000 Tabletten, die 3 mg Trandolapril und 50 mg Verapamil enthalten, werden wie folgt hergestellt:
Trandolapril 3 g
Verapamil 50 g
Maisstärke 130 g
Gelatine 8,0 g
Mikrokristalline Cellulose 2,0 g
Magnesiumstearat 2,0 g

Die beiden Wirkstoffe werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das so hergestellte Granulat wird zu 1000 Tabletten verpreßt, wobei jede Tablette 3 mg Trandolapril und 50 mg Verapamil enthält.

## Patentansprüche (Patentansprüche für folgende(n) Vertragsstaat(en): AT, BE, CH, DE, DK, FR, GB, IT, LI, LU, NL, SE)

1. Pharmazeutische Zubereitung, enthaltend Trandolapril und Verapamil oder deren physiologisch verträglichen Salze.

## Patentansprüche (Patentansprüche für folgende(n) Vertragsstaat(en): ES, GR)

1. Verfahren zur Herstellung einer pharmazeutischen Zubereitung enthaltend Trandolapril und Verapamil oder deren physiologisch verträglichen Salze, dadurch gekennzeichnet, daß man diese Komponenten zusammen mit weiteren Hilfsstoffen in eine geeignete Darreichungsform bringt.

## Claims (Claims for the following Contracting State(s): AT, BE, CH, DE, DK, FR, GB, IT, LI, LU, NL, SE)

1. A pharmaceutical preparation containing trandolapril and verapamil or their physiologically tolerable salts.

## Claims (Claims for the following Contracting State(s): ES, GR)

1. A process for the production of a pharmaceutical preparation containing trandolapril and verapamil or their physiologically tolerable salts, which comprises bringing these components into a suitable administration form together with other auxiliaries.

## Revendications (Revendications pour l'(les) Etat(s) contractant(s) suivant(s): AT, BE, CH, DE, DK, FR, GB, IT, LI, LU, NL, SE)

1. Composition pharmaceutique, contenant du trandolapril et du vérapamil ou leurs sels physiologiquement acceptables.

## Revendications (Revendications pour l'(les) Etat(s) contractant(s) suivant(s): ES, GR)

1. Procédé pour la préparation d'une composition pharmaceutique, contenant du trandolapril et du vérapamil ou leurs sels physiologiquement acceptables, caractérisé en ce que l'on met sous une forme d'administration appropriée ces composants, conjointement avec d'autres adjuvants.
